# EUROPEAN PATENT APPLICATION

(11) **EP 4 510 141 A1**
(43) Date of publication of application: **19.02.2025**
(21) Application number: 24194399.2
(22) Date of filing: 13.08.2024
(51) Int. Cl.: G16H 20/00, G16H 50/20

(54) **METHOD OF ADAPTIVE DIGITAL TREATMENT BASED ON USER'S PHENOTYPE AND APPARATUS FOR PERFORMING THE METHOD**

(30) Priority: 14.08.2023 KR 20230106200
(71) Applicant: WELT Corp., Ltd, Seocho-gu Seoul 06628 (KR)
(72) Inventor: SHIM, Hwa Jin, 07229 Seoul (KR); KIM, Hye Ryong, 12997 Gyeonggi-do (KR); KANG, Seong Ji, 06366 Seoul (KR); ROH, Hye Kang, 05507 Seoul (KR)
(74) Representative: AWA Sweden AB

(57) **Abstract**

The present invention relates to a method of adaptive digital treatment based on a phenotype of a user and an apparatus for performing the method. The method includes receiving, by an apparatus for phenotype-based digital treatment, data related to a biomarker of a user; determining, by the apparatus for phenotype-based digital treatment, a phenotype of the user based on the biomarker, and performing, by the apparatus for phenotype-based digital treatment, digital treatment based on the phenotype.

## Description

### CROSS-REFERENCE TO RELATED APPLICATION

This application claims priority to and the benefit of Korean Patent Application No. 10-2023-0106200, filed on August 14, 2023, the disclosure of which is incorporated herein by reference in its entirety.

### BACKGROUND

### 1. Field of the Invention

The present invention relates to a method of adaptive digital treatment based on a phenotype of a user and an apparatus for performing the method. More specifically, the present invention relates to a method of adaptive digital treatment based on a phenotype of a user that is capable of classifying the phenotype of the user based on a biomarker and performing digital treatment on the user through the classified phenotype, and an apparatus for performing the method.

### 2. Discussion of Related Art

With the development of various smart technologies, data related to personal daily activities is being recorded, and personal lives may be managed more efficiently based on the recorded data. Among the various smart technologies, health-related data logging has gained attention as interest in health has increased. Many users are already generating and utilizing various health-related data, such as exercise, diet, and sleep of users, through user devices, such as smartphones and wearable devices. This represents a shift from the past in which health-related data was generated and managed only by medical institutions to a situation in which users have begun to generate and manage their own health-related data through user devices, such as smartphones or wearable devices.

Health-related data logging is often performed through wearable devices. A wearable device is a user device that is carried or attached to a user's body. With advancements of the Internet of Things (IoT) and the like, wearable devices are being widely used to collect health-related data. Wearable devices may collect information about a user's physical changes and environmental data surrounding the user, and provide advice required for the user's health based on the collected data.

Health-related data of a user may include user biomarkers, and research is being conducted on a method of adaptively performing medical prescriptions for users based on health-related data of users.

The related art is disclosed in Korean Registered Patent No. 10-2425479.

### SUMMARY OF THE INVENTION

The present invention aims to resolve all of the issues described above.

The present invention is directed to providing a user with a digital treatment by determining a phenotype of the user for each category based on a set biomarker of the user and setting an adaptive digital treatment algorithm according to the phenotype of the user.

In addition, the present invention is directed to determining a phenotype of a user by considering the relationship between phenotypes of the user and designing a user treatment protocol by considering the phenotype of the user.

A representative configuration of the present invention for achieving the above objects is as follows.

According to an aspect of the present invention, there is provided a method of adaptive digital treatment based on a phenotype of a user, the method comprising: receiving, by an apparatus for phenotype-based digital treatment, data related to a biomarker of a user; determining, by the apparatus for phenotype-based digital treatment, a phenotype of the user based on the biomarker; and performing, by the apparatus for phenotype-based digital treatment, digital treatment based on the phenotype.

Meanwhile, the phenotype is generated to correspond to a category, and the category is set based on a disease to set a digital treatment algorithm for the digital treatment.

Further, the category and the phenotype adaptively change based on a digital treatment result of the digital treatment.

According to another aspect of the present invention, there is provided an apparatus for phenotype-based digital treatment for performing an adaptive digital treatment based on a phenotype of a user, the apparatus being implemented to receive data related to a biomarker of a user, determine a phenotype of the user based on the biomarker; and perform digital treatment based on the phenotype.

Meanwhile, the phenotype is generated to correspond to a category, and the category is set based on a disease to set a digital treatment algorithm for the digital treatment.

Further, the category and the phenotype adaptively change based on a digital treatment result of the digital treatment.

### BRIEF DESCRIPTION OF THE DRAWINGS

FIG. 1 is a conceptual diagram illustrating an apparatus for phenotype-based digital treatment for performing adaptive digital treatment based on a phenotype of a user according to an embodiment of the present invention.
FIG. 2 is a conceptual diagram illustrating a phenotype for digital treatment according to an embodiment of the present invention.
FIG. 3 is a conceptual diagram illustrating a method of setting a digital treatment algorithm according to a phenotype according to an embodiment of the present invention.
FIG. 4 is a conceptual diagram illustrating a method of determining a digital treatment sequence according to an embodiment of the present invention.
FIG. 5 is a conceptual diagram illustrating a method of digital treatment according to a category and a phenotype according to an embodiment of the present invention.
FIG. 6 is a conceptual diagram illustrating a method of determining categories and phenotypes according to an embodiment of the present invention.
FIG. 7 is a conceptual diagram illustrating a method of changing phenotypes according to a category adjustment according to an embodiment of the present invention.

### DETAILED DESCRIPTION OF EXEMPLARY EMBODIMENTS

The detailed description of the present invention will be made with reference to the accompanying drawings showing examples of specific embodiments of the present invention. These embodiments will be described in detail such that the present invention can be performed by those skilled in the art. It should be understood that various embodiments of the present invention are different but are not necessarily mutually exclusive. For example, a specific shape, structure, and characteristic of an embodiment described herein may be implemented in another embodiment without departing from the scope and spirit of the present invention. In addition, it should be understood that a position or arrangement of each component in each disclosed embodiment may be changed without departing from the scope and spirit of the present invention. Accordingly, there is no intent to limit the present invention to the detailed description to be described below. The scope of the present invention is defined by the appended claims and encompasses all equivalents that fall within the scope of the appended claims. Like reference numerals refer to the same or like elements throughout the description of the figures.

Hereinafter, in order to enable those skilled in the art to practice the present invention, exemplary embodiments of the present invention will be described in detail with reference to the accompanying drawings.

Hereinafter, embodiments of the present invention will be described for the sake of convenience of description with an apparatus for phenotype-based digital treatment for treating insomnia by way of an example. However, the embodiments of the present invention may employ phenotype-based digital treatment algorithms for various other diseases as well as insomnia, and such embodiments may also be included within the scope of the present invention.

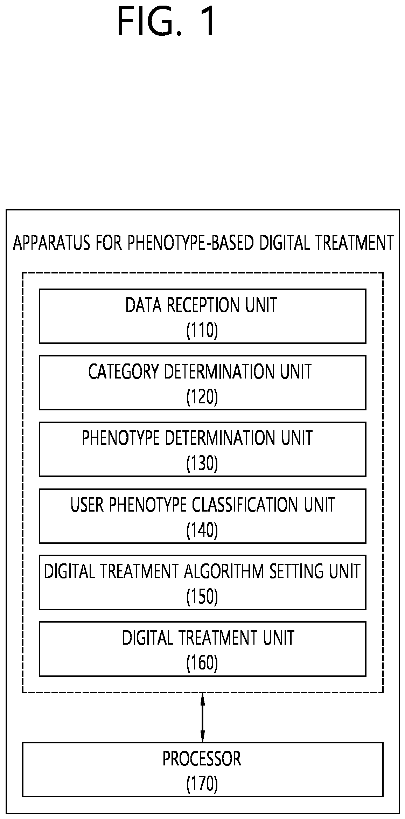

FIG. 1 is a conceptual diagram illustrating an apparatus for phenotype-based digital treatment for performing adaptive digital treatment based on a phenotype of a user according to an embodiment of the present invention.

In FIG. 1, an apparatus for phenotype-based digital treatment may include a data reception unit 110, a category determination unit 120, a phenotype determination unit 130, a user phenotype classification unit 140, a digital treatment algorithm setting unit 150, a digital treatment unit 160, and a processor 170.

Referring to FIG. 1, the data reception unit 110 may be implemented to receive data for providing a user with digital treatment services, such as a biomarker of the user. The biomarker of the user may be collected through various media, such as smartphone sensors and applications, as well as wearable devices.

The category determination unit 120 may be implemented to determine categories for determining phenotypes. In the present invention, categories may be set, and category-specific phenotypes may be determined. For example, insomnia may be classified into a misperception category, a subjective/objective insomnia category, an insomnia symptom, and the like.

The category determination unit 120 may determine a category for digital treatment for the user. Categories may be changed (added, deleted, integrated, etc.) according to changes in the digital treatment algorithm.

The phenotype determination unit 130 may be implemented to determine a phenotype corresponding to a category corresponding to the category determination unit 120. For example, as category-specific phenotypes for the misperception category, phenotypes of a user may be determined as negative sleep misperception (moderately misperceptive), negative sleep misperception (highly misperceptive), no sleep misperception, positive sleep misperception, etc., based on a biomarker of the user.

As another example, as phenotypes for the subjective/objective insomnia category, phenotypes of a user may be determined to objective insomnia, subjective insomnia, and the like based on a biomarker of the user.

The phenotype determination unit 130 may be classified according to criteria set for each category. The phenotype determination unit 130 may be divided by considering the set criteria (e.g., numerical values, scores, etc. of set parameters). In the subjective/objective insomnia category, phenotypes of a user may be determined based on parameters, such as sleep onset latency (SOL), wake after sleep onset (WASO), number of awakenings (NOA)/Hr, sleep efficiency (SE), and the like, as subjective insomnia when the biomarker satisfies a first condition of the set parameters, and as objective insomnia when the biomarker satisfies a second condition of the set parameters.

The phenotype determination unit 130 may change (add, delete, integrate) the phenotype according to the digital treatment algorithm.

The user phenotype classification unit 140 may be implemented to classify the phenotype of a user based on a biomarker of the user. When the biomarker of the user is input, the phenotype of the user corresponding to a category may be determined based on the biomarker of the user. For example, the phenotype of a user may be determined as negative sleep misperception (moderately misperceptive) for the misperception category, and objective insomnia for the subjective/objective insomnia category.

The phenotype of the user may be expressed in a code format. For example, a first category, a second category, a third category, and a fourth category may be assigned individual positions, and when the phenotype of the first category is A1, the phenotype of the second category is B2, the phenotype of the third category is C3, and the phenotype of the fourth category is D1, the phenotype of the user may be expressed as "A1B2/C3/D1."

The digital treatment algorithm setting unit 150 may be implemented to set a digital treatment algorithm according to the phenotype of the user. When the phenotype of the user is "A1/B2/C3/D1," a digital treatment sequence, a digital treatment content provision sequence, a digital treatment activity priority, and the like that suit the phenotype of the user may be determined.

The digital treatment algorithm setting unit 150 may provide a digital treatment algorithm in consideration of the phenotype of the user and change or adjust the digital treatment algorithm based on a digital treatment result (or digital treatment feedback) for the user.

The processor 170 may control operations of the data reception unit 110, the category determination unit 120, the phenotype determination unit 130, the user phenotype classification unit 140, the digital treatment algorithm setting unit 150, and the digital treatment unit 160.

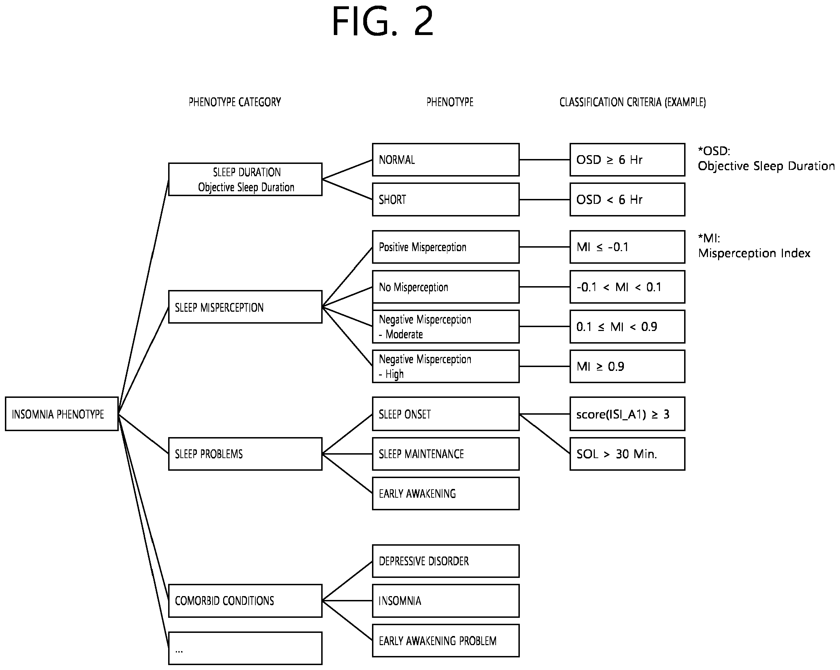

FIG. 2 is a conceptual diagram illustrating a phenotype for digital treatment according to an embodiment of the present invention.

In FIG. 2, an example of a method of dividing phenotypes of a user for digital treatment is illustrated. In particular, in FIG. 2, phenotypes of insomnia are illustrated, but phenotypes of various other diseases as well as insomnia may be disclosed in the same manner, and the corresponding embodiments may also be included within the scope of the present invention.

Referring to FIG. 2, categories 200 for determining a phenotype 220 of insomnia may be divided into 1) an objective/subjective category, 2) a sleep duration category, 3) a sleep problem category, 4) a comorbidity category, 5) a hyperarousal category, and the like.

The objective/subjective category may have objective insomnia and subjective insomnia as the phenotypes 220.

The sleep duration category may have long, normal, and short as the phenotypes 220.

The sleep problem category may include a sleep onset problem, a sleep maintenance problem, and an early awakening problem as the phenotypes 220.

The comorbidity category may include a depressive disorder, an anxiety disorder, and a bipolar disorder as the phenotypes 220.

The hyperarousal category may include cognitive/emotional hyperarousal and physiological hyperarousal as the phenotypes 220.

As described above, the categories 200 and the phenotypes 220 for disease may be defined, and the categories 200 and the phenotypes 220 may be changed (integrated, deleted, added, etc.) based on a user's digital treatment results (or digital treatment feedback).

Additionally, according to an embodiment of the present invention, the relationships of the categories 200 and the phenotypes 220 may be defined. The categories 200 may have a dependent or independent relationship, and the phenotypes 220 may also be defined as having a dependent or independent relationship.

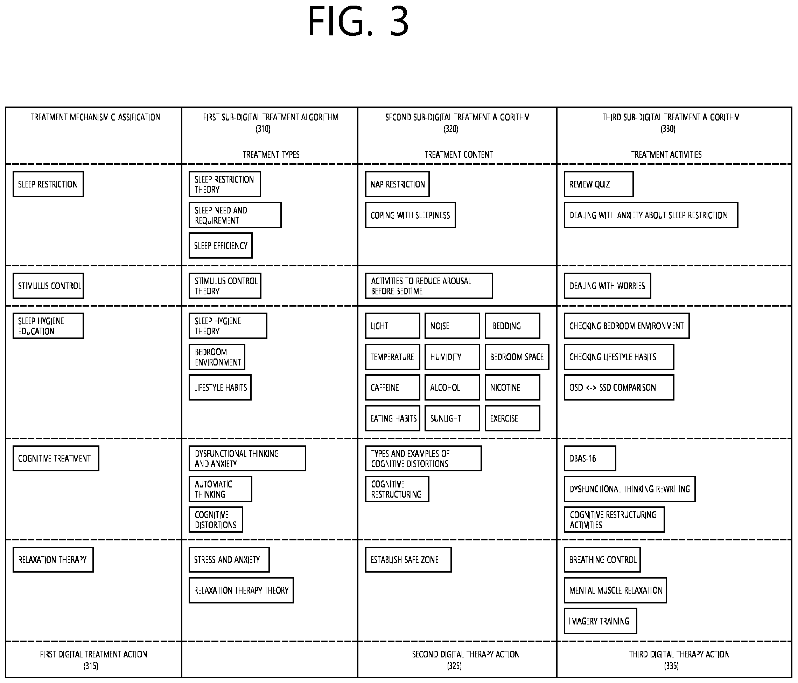

FIG. 3 is a conceptual diagram illustrating a method of setting a digital treatment algorithm according to a phenotype according to an embodiment of the present invention.

In FIG. 3, a method of determining a phenotype of a user and setting a digital treatment algorithm based on the phenotype of the user is disclosed.

Referring to FIG. 3, a method of determining a phenotype of a user based on a biomarker of the user and adaptively providing treatment types, treatment content (or treatment articles), and treatment activities to the user as a digital treatment algorithm is disclosed.

The treatment type, the treatment content (or the treatment article), and the treatment activity may be sub-digital treatment algorithms that constitute a digital treatment algorithm. The digital treatment algorithm may include a plurality of sub-digital treatment algorithms (e.g., a first sub-digital treatment algorithm to an n^{th} sub-digital treatment algorithm). A dependent relationship or an independent relationship may be set between the sub-digital treatment algorithms.

Each of the sub-digital treatment algorithms may include a plurality of digital treatment actions. For example, a first sub-digital treatment algorithm 310 may include a first digital treatment action-1 to a first digital treatment action-n. A second sub-digital treatment algorithm 320 may include a second digital treatment action-1 to a second digital treatment action-n.

As a more specific example, a first sub-digital treatment algorithm 310 may be an algorithm for direct sleep treatment. The first sub-digital treatment algorithm 310 may include sleep restriction, stimulus control, sleep hygiene, cognitive treatment, relaxation treatment, and the like as a first digital treatment action 315.

The sleep restriction is a treatment that increases sleep efficiency by limiting the time spent in bed. The stimulus control is a behavioral treatment to break conditioned arousal in bed and re-associate the bed and bedroom space with sleep rather than arousal. The cognitive restructuring is a treatment method that breaks the vicious cycle of insomnia caused by distorted cognitions. The relaxation therapy is a method that promotes physical and mental relaxation, such as breath control, progressive muscle relaxation, imagery training, and mindfulness meditation. The sleep hygiene education involves teaching guidelines and recommendations to form healthy sleep habits and improve sleep quality by improving habitual/environmental factors.

A second sub-digital treatment algorithm 320 may be an algorithm for providing sleep treatment content. The second sub-digital treatment algorithm 320 may include nap content, postprandial drowsiness content, cognitive distortion content, and the like as a second digital treatment action 325.

A third sub-digital treatment algorithm 330 may be an algorithm for providing sleep treatment activities. The third sub-digital treatment algorithm 330 may include OX quizzes, letting go of thoughts, sleep hygiene action policy, bedroom environment checks, relaxation breathing, and the like as a third digital treatment action 335.

According to an embodiment of the present invention, a dependent relationship may be set between sub-digital treatment algorithms. Once a main digital treatment sequence based on a specific sub-digital treatment algorithm is determined, a sub-digital treatment sequence based on another dependent sub-digital treatment algorithm that follows the main digital treatment sequence may be determined. Thereafter, digital treatment actions based on the main digital treatment sequence and the sub-digital treatment sequence may be provided to the user.

For example, based on sleep restriction, which is the first digital treatment action 315 of the first sub-digital treatment algorithm 310, a dependent relationship may be set such that nap content, postprandial drowsiness content, and content related to worries about sleep restriction of the second sub-digital treatment algorithm 320, and OX quizzes of the third sub-digital treatment algorithm 330 are linked.

The main digital treatment sequence may be preferentially determined based on the first sub-digital treatment algorithm 310. For example, the main digital treatment sequence for sleep restriction, stimulus control, sleep hygiene, cognitive treatment, and relaxation corresponding to the first digital treatment action 315 may be determined. Once the main digital treatment sequence is determined based on the first sub-digital treatment algorithm 310, a sub-digital treatment sequence, which is a sequence of providing digital actions corresponding to other sub-digital treatment algorithms (the second sub-digital treatment algorithm 320 and the third sub-digital treatment algorithm 330) dependent on the first sub-digital treatment algorithm 310 may be determined.

The main digital treatment sequence and/or the sub-digital treatment sequence may be determined based on the phenotype and/or the biomarker of the user.

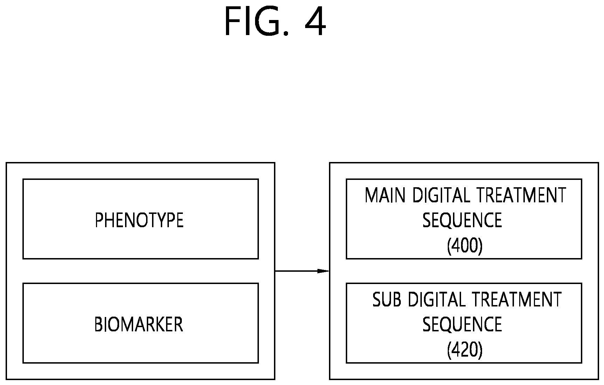

FIG. 4 is a conceptual diagram illustrating a method of determining a digital treatment sequence according to an embodiment of the present invention.

In FIG. 4, a method of determining a digital treatment sequence based on a biomarker of a user is disclosed.

Referring to FIG. 4, a phenotype may be determined based on a biomarker of a user, and a main digital treatment sequence 400 and a sub-digital treatment sequence 420 according to the phenotype may be determined. The biomarker may be obtained through wearable devices, user records (e.g., sleep diaries), questionnaires, and the like.

For example, when a user has a phenotype of A1/B2/C3/D1, the main digital treatment sequence 400 may be determined in a sequence of sleep restriction, stimulus control, sleep hygiene, cognitive treatment, and relaxation. For example, when a user has a phenotype of A2/B1/C1/D2, the main digital treatment sequence 400 may be determined in a sequence of cognitive treatment, relaxation, stimulation control, sleep restriction, and sleep hygiene. After the main digital treatment sequence 400 is determined, the sub-digital treatment sequence 420 of the user may also be determined based on the phenotype, and digital treatment actions may be provided to the user.

Additionally, according to an embodiment of the present invention, the main digital treatment sequence 400 and the sub-digital treatment sequence 420 of the user may be determined by considering the biomarker of the user. Answers to the questionnaire provided to the user may be the biomarker of the user, and the main digital treatment sequence 400 and the sub-digital treatment sequence 420 of the user may be determined based on the answers to the questionnaire provided to the user.

Digital treatment actions (or interventions) for each questionnaire provided to the user and weights for the digital treatment actions may be set, and a main digital treatment sequence to be preferentially provided to the user and a sub-digital treatment sequence may be determined based on the answers to the questionnaire. By applying the weights of the answers to the questionnaire, first scores for the digital treatment actions corresponding to the main digital treatment sequence and second scores for the digital treatment actions corresponding to the sub-digital treatment sequence may be determined, and the main digital treatment sequence and the sub-digital treatment sequence may be determined based on the first scores and the second scores.

According to an embodiment of the present invention, the main digital treatment sequence and the sub-digital treatment sequence may be determined by combining the phenotype and the biomarker of the user. For example, the main digital treatment sequence may be determined based on the phenotype, and the sub-digital treatment sequence may be determined based on the biomarker.

Additionally, the weights and the scores for determining the main digital treatment sequence and the sub-digital treatment sequence may be preferentially set based on a default treatment algorithm, and then adjusted based on digital treatment results (or digital treatment feedback) for the user. For example, the weights and the scores may be set such that a specific digital treatment action should be included in a specific treatment sequence and that there is no overlap between specific digital treatment actions. Thereafter, the weights and the scores may be adjusted based on digital treatment results (or digital treatment feedback) for the user, and the default treatment algorithm may be changed to an algorithm that reflects the digital treatment results.

In addition, the speed and cycle of digital treatment may be adjusted considering the phenotype and biomarker of the user, and the cycle for tracking the biomarker of the user may also be adjusted.

Biomarkers may be obtained through wearable devices, user records (e.g., sleep diaries), questionnaires, and the like.

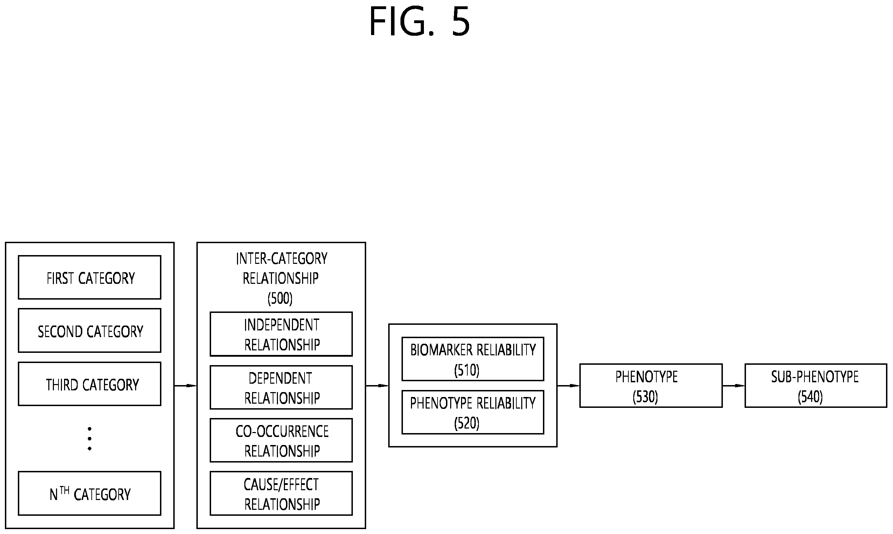

FIG. 5 is a conceptual diagram illustrating a method of digital treatment according to a category and a phenotype according to an embodiment of the present invention.

In FIG. 5, a method of performing digital treatment considering relationships between categories and/or phenotypes is disclosed.

Referring to FIG. 5, an independent relationship and a dependent relationship may be set as an inter-category relationship 500. Alternatively, a co-occurrence relationship or a cause/effect relationship may be present in the inter-category relationship 500. A digital treatment algorithm may be set considering the inter-category relationship 500. For example, in the category for insomnia treatment, the subjective/objective insomnia category may have a co-occurrence relationship with the sleep misperception category, and the cognitive/emotional hyperarousal category and the subjective/objective insomnia category may have a cause/effect relationship. Such an inter-category relationship is only an example, and the inter-category relationship may be set in various types.

Based on the inter-category relationship 500, a phenotype 530 of a user considering a biomarker of the user may be determined. For example, a phenotype reliability 520 for the phenotype 530 of the user and a biomarker reliability 510 of the user may be determined by considering the inter-category relationship 500. For example, in the categories having a co-occurrence relationship, when the phenotype 530 of the user is contradictory or the biomarkers of the user have a contradictory relationship, the phenotype reliability 520 and the biomarker reliability 510 may be adjusted.

Additionally, based on the inter-category relationship 500, the phenotype 530 may be divided into a plurality of sub-phenotypes 540. For example, when the phenotype 530 of the user is A1/B2/C3/D1, the phenotype 530 may be divided into a sub-phenotype 1 A1/B2 and a sub-phenotype 2 C3/D1.

The phenotype reliability 520 for each phenotype 530 may be determined, and based on the phenotype reliability 520 for each phenotype 530, a sub-phenotype 540 may be determined for a phenotype 530 having a phenotype reliability 520 greater than a threshold. Only a phenotype having a reliability higher than or equal to a threshold reliability that is set for each phenotype 530 may be extracted, the sub-phenotype 540 may be expressed, and based on the sub-phenotype 540, a digital treatment algorithm may first be determined.

Alternatively, digital treatment algorithms may be set for each of a plurality of sub-phenotypes 540, and a main digital treatment sequence and a sub-digital treatment sequence may be determined for each sub-phenotype 540. Between sub-phenotypes 540, a sub-phenotype 540 having a faster treatment effect or requiring faster treatment may be set as a higher priority sub-phenotype 540, and a digital treatment algorithm based on the corresponding sub-phenotype 540 may be preferentially performed. When the phenotype of a user is A1/B2/C3/D1, the phenotype may be separated into sub-phenotype 1 A1/B2 and sub-phenotype 2 C3/D1. A digital treatment algorithm based on the sub-phenotype 1 A1/B2 may be preferentially performed, and digital treatment may be performed through a main digital treatment sequence and a sub-digital treatment sequence that are set based on the sub-phenotype 1 A1/B2. Thereafter, a digital treatment algorithm based on the sub-phenotype 2 C3/D1 may be performed, and digital treatment may be performed through a main digital treatment sequence and a sub-digital treatment sequence that are set based on the sub-phenotype 2 C3/D1.

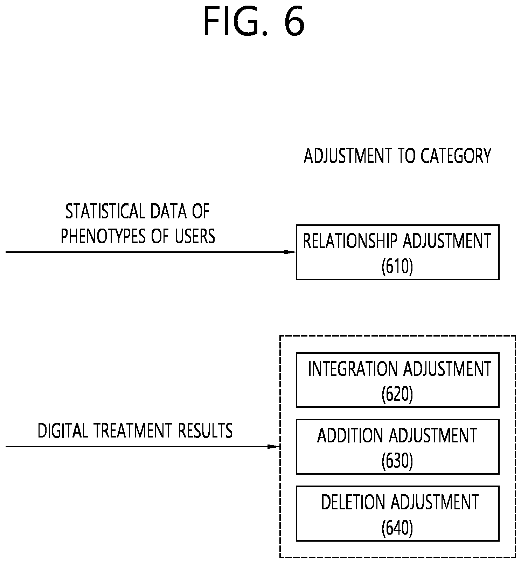

FIG. 6 is a conceptual diagram illustrating a method of determining categories and phenotypes according to an embodiment of the present invention.

In FIG. 6, a method of adjusting categories for determining a digital treatment algorithm for treatment of a user's disease is disclosed.

Referring to FIG. 6, an adjustment to categories may be classified into a relationship adjustment (610), an integration adjustment (620), an addition adjustment (630), and a deletion adjustment (640). According to the adjustments to the categories, the phenotypes may also be adjusted, and according to the adjustments to the phenotypes, the digital treatment algorithm may also be adjusted.

The relationship adjustment 610 involves adjusting the relationships between categories. The existing relationships between categories may be set as an independent relationship, a dependent relationship, a co-occurrence relationship, a cause/effect relationship, and the like. The relationship adjustment may be performed by considering the relationship between the phenotypes of users. To set the relationships between the phenotypes of users, statistical data between the phenotypes may be extracted, a relation may be extracted based on the statistical data (e.g., correlation) between the phenotypes, and the relation may be corrected based on the phenotypes of actual users. For example, a default relationship between Category A and Category B may be set as an independent relationship. However, when statistical data is extracted as indicating that there is a dependent relationship between a specific phenotype of Category A and a specific phenotype of Category B, the relationship between Category A and Category B may be converted from an independent relationship to a dependent relationship.

According to an embodiment of the present invention, there is an established standard statistical threshold for setting a default relationship between categories defined for each disease. When the standard statistical threshold is exceeded, the relationship between categories may be adjusted.

The integration adjustment 620, the addition adjustment 630, and the deletion adjustment 640 may be adjusted based on digital treatment results (or digital treatment feedback).

The integration adjustment 620 may be an adjustment of performing integration between categories. The integration between categories may be performed by considering the difference between a digital treatment result considering Category A and a digital treatment result considering Category B. For the integration adjustment 620 between categories, a first test user group that performs treatment through integration between different categories may be set, and when the difference between digital treatment results of the test user group and digital treatment results for each category is less than or equal to an integration adjustment threshold difference value (e.g., disease improvement less than or equal to the integrated adjustment threshold difference value), the integration adjustment 620 between categories may be performed.

The addition adjustment 630 may be an adjustment of adding a category. The addition of a category may be performed by dividing the characteristics of a specific category into a plurality of sub-categories and considering the differences between digital treatment results for each sub-category. For the addition adjustment (630) for the category, a plurality of second test user groups may be set by dividing a specific category into a plurality of sub-categories, and when the difference between digital treatment results of each of the plurality of second test user groups is greater than or equal to an addition adjustment threshold difference value (e.g., disease improvement greater than or equal to the addition adjustment threshold difference), the addition adjustment of categories may be performed.

The deletion adjustment 640 may be an adjustment of deleting a category. The deletion of a category may be performed by considering the difference between the digital treatment results before and after excluding a specific category. For the deletion adjustment 640 for a category, a third test user group for which a specific category is deleted may be set, and when the difference in digital treatment results of the third test user group is less than or equal to a deletion adjustment threshold difference value (e.g., disease improvement less than or equal to the deletion adjustment threshold difference value), the deletion adjustment 640 of categories may be performed.

The integration adjustment threshold difference value, the addition adjustment threshold difference value, and the deletion adjustment threshold difference value may be set for each disease category, and the size of the integration adjustment threshold difference value, the addition adjustment threshold difference value, and the deletion adjustment threshold difference value may change continuously depending on the timing of changes after a default category setting.

More specifically, for categories set based on the existing clinical data, the integration adjustment threshold difference value, the addition adjustment threshold difference value, and the deletion adjustment threshold difference value may be set to be relatively larger. Conversely, for categories set without clinical data, the integration adjustment threshold difference value, the addition adjustment threshold difference value, and the deletion adjustment threshold difference value may be set to be relatively smaller.

In addition, after the default category setting, the addition adjustment threshold difference value and the deletion adjustment threshold difference value may be set to be relatively smaller, and as the adjustments proceed to an n^{th} adjustment from a primary adjustment, the addition adjustment threshold difference value and the deletion adjustment threshold difference value may be set relatively larger.

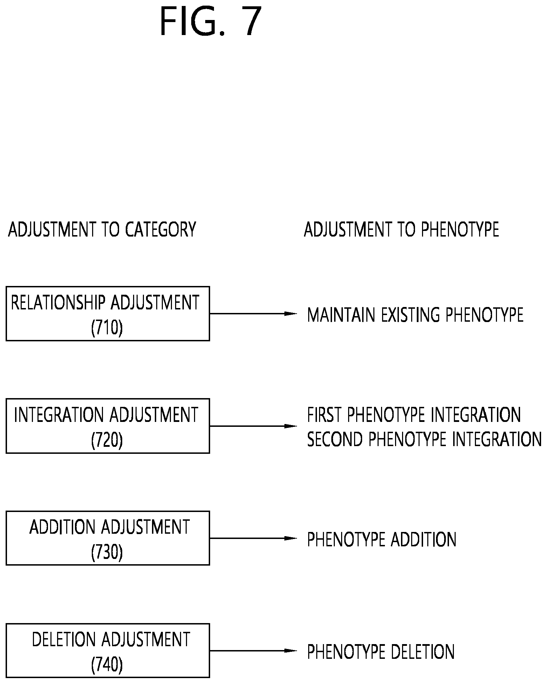

FIG. 7 is a conceptual diagram illustrating a method of changing phenotypes according to a category adjustment according to an embodiment of the present invention.

In FIG. 7, a method of changing the phenotype when the category is adjusted is disclosed.

Referring to FIG. 7, the phenotype may vary according to category adjustments.

When the category adjustment is a relationship adjustment (710), the existing phenotype may be used without a separate change in the phenotype. However, an adjustment to sub-phenotypes determined based on the relationship between categories may occur, and the digital treatment algorithm may change according to the adjustment to the sub-phenotypes.

When the category adjustment is an integration adjustment (720), integration into an existing phenotype due to integration of two categories or integration after generation of a new phenotype may be achieved. The integration into the existing phenotype may be first phenotype integration, and the integration after generation of the new phenotype may be second phenotype integration.

The first phenotype integration and the second phenotype integration may be determined by considering the relationship between a category that is the target of integration and other categories.

In the first phenotype integration, a plurality of phenotypes corresponding to a plurality of categories may be integrated into a single phenotype corresponding to a single category. In this case, a phenotype that is determined as a single phenotype by the integration may be determined as a phenotype corresponding to a category having relatively more inter-category relationships with other categories, by considering the relationships between the two categories and other categories. For example, when Category A and Category B are integrated, a phenotype corresponding to a category having more independent relationships, dependent relationships, co-occurrence relationships, and cause/effect relationships with other categories by considering the relationships of each of Category A and Category B with other categories may be used as an integration phenotype.

The second phenotype integration may involve generating a new phenotype, and corresponds to a method of defining and setting a new phenotype when two categories do not have a separate relationship with other categories.

When the category adjustment is an addition adjustment (730), a phenotype according to the addition of a new category may be added. An adjustment of a digital treatment algorithm according to the addition of phenotype may be achieved. The adjustment of the digital treatment algorithm may be a new setting of a digital treatment algorithm corresponding to a new phenotype.

When the category adjustment is a deletion adjustment (740), a deletion of a phenotype according to the deletion of the category may be achieved. An adjustment of a digital treatment algorithm may be achieved by deleting a digital treatment algorithm corresponding only to the deleted phenotype among digital treatment algorithms corresponding to the deleted phenotype while maintaining a digital treatment algorithm corresponding to even other phenotypes.

The embodiments of the present invention described above may be implemented in the form of program instructions that can be executed through various computer units and recorded on computer readable media. The computer readable media may include program instructions, data files, data structures, or combinations thereof. The program instructions recorded on the computer readable media may be specially designed and prepared for the embodiments of the present invention or may be available instructions well known to those skilled in the field of computer software. Examples of the computer readable media include magnetic media such as a hard disk, a floppy disk, and a magnetic tape, optical media such as a compact disc read only memory (CD-ROM) and a digital video disc (DVD), magneto-optical media such as a floptical disk, and a hardware device, such as a ROM, a RAM, or a flash memory, that is specially made to store and execute the program instructions. Examples of the program instruction include machine code generated by a compiler and high-level language code that can be executed in a computer using an interpreter and the like. The hardware device may be configured as at least one software module in order to perform operations of embodiments of the present invention and vice versa.

While the present invention has been described with reference to specific details such as detailed components, specific embodiments and drawings, these are only examples to facilitate overall understanding of the present invention and the present invention is not limited thereto. It will be understood by those skilled in the art that various modifications and alterations may be made.

Therefore, the spirit and scope of the present invention are defined not by the detailed description of the present invention but by the appended claims, and encompass all modifications and equivalents that fall within the scope of the appended claims.

## Claims

1. A method of adaptive digital treatment based on a phenotype of a user, the method comprising:
receiving, by an apparatus for phenotype-based digital treatment, data related to a biomarker of a user;
determining, by the apparatus for phenotype-based digital treatment, a phenotype of the user based on the biomarker; and
performing, by the apparatus for phenotype-based digital treatment, digital treatment based on the phenotype.

2. The method of claim 1, wherein the phenotype is generated to correspond to a category, and the category is set based on a disease to set a digital treatment algorithm for the digital treatment.

3. The method of claim 2, wherein the category and the phenotype adaptively change based on a digital treatment result of the digital treatment.

4. An apparatus for phenotype-based digital treatment for performing an adaptive digital treatment based on a phenotype of a user, the apparatus being implemented to:
receive data related to a biomarker of a user;
determine a phenotype of the user based on the biomarker; and
perform digital treatment based on the phenotype.

5. The apparatus of claim 4, wherein the phenotype is generated to correspond to a category, and the category is set based on a disease to set a digital treatment algorithm for the digital treatment.

6. The apparatus of claim 5, wherein the category and the phenotype adaptively change based on a digital treatment result of the digital treatment.
